# EUROPEAN PATENT SPECIFICATION

(11) **EP 0 424 445 B1**
(45) Date of publication and mention of the grant of the patent: **06.09.1995**
(21) Application number: 89908293.7
(22) Date of filing: 11.07.1989
(51) Int. Cl.: C07C 2/58

(54) **HETEROGENEOUS ISOPARAFFIN/OLEFIN ALKYLATION PROCESS**
HETEROGENES ISOPARAFFIN/OLEFIN-ALKYLIERUNGSVERFAHREN
PROCEDE HETEROGENE D'ALKYLATION D'ISOPARAFFINE/OLEFINE

(30) Priority: 15.07.1988 US 219130; 15.07.1988 US 219527
(43) Date of publication of application: 02.05.1991
(73) Proprietor: MOBIL OIL CORPORATION, New York New York 10017 (US)
(72) Inventor: CHOU, Tai-Sheng, Pennington, NJ 08534 (US); HUSS, Albin, Jr., Chadds Ford, PA 19317 (US); KENNEDY, Clinton, Robert, West Chester, PA 19382 (US); KURTAS, Robert, Steve, Sewell, NJ 08080 (US)
(74) Representative: Colmer, Stephen Gary
(86) International application number: US8903002
(87) International publication number: WO9000533

(56) References cited:
- GB-A- 546 406
- US-A- 2 363 116
- US-A- 2 450 764
- US-A- 3 800 003
- US-A- 4 384 161
- US-A- 4 774 364

## Description

The present invention relates to a heterogeneous process for alkylating an isoparaffin with olefin to provide an alkylate product useful as an ocatane enhancer for gasoline.

As a result of restrictions in the use of tetraethyl lead as an octane-improving additive for gasoline, not only has the production of unleaded gasoline increased but the octane number specification of all grades of gasoline has increased as well. Isoparaffin-olefin alkylation is a key route to the production of highly branched paraffin octane enhancers for blending into gasolines to provide the required octance increase.

Alkylation involves the addition of an alkyl group to an organic molecule. Thus, an isoparaffin can be reacted with an olefin to provide an isoparaffin of higher molecular weight. Industrially, alkylation often involves the reaction of a C₂-C₅ olefin with isobutane in the presence of an acidic catalyst to produce a so-called alkylate. Alkylates are valuable blending components for the manufacture of premium gasolines due to their high octane ratings.

In the past, alkylation processes have included the use of hydrofluoric acid or sulfuric acid as catalysts under controlled temperature conditions. Low temperatures are utilized in the sulfuric acid process to minimize the undesirable side reaction of olefin polymerization and the acid strength is generally maintained at 88-94 percent by the continuous addition of fresh acid and the continuous withdrawal of spent acid. The hydrofluoric acid process is less temperature-sensitive and the acid is easily recovered and purified.

However, the hydrofluoric acid and sulfuric acid alkylation processes have inherent drawbacks including environmental concerns, acid consumption and disposal of corrosive materials. With increasing demands for octane and increasing environmental concerns, there is a need to develop an alkylation process based on a solid catalyst system.

Crystalline zeolites, have been widely investigated for use as alkylation catalysts. For example, U.S. Patent No. 3,251,902 describes the use of a fixed bed of ion-exchanged crystalline aluminosilicate having a reduced number of available acid sites for the liquid phase alkylation of C₄-C₂₀ branched-chain paraffins with C₂-C₁₂ olefins. The patent further discloses that the C₄-C₂₀ branched-chain paraffin should be allowed to substantially saturate the crystalline aluminosilicate before the olefin is introduced into the alkylation reactor.

U.S. Patent No. 3,549,557 describes the alkylation of isobutane with C₂-C₃ olefins using certain crystalline aluminosilicate zeolite catalysts in a fixed, moving or fluidized bed system, the olefin being preferably injected at various points in the reactor.

U.S. Patent No. 3,644,565 discloses the alkylation of a paraffin with an olefin in the presence of catalyst comprising a Group VII noble metal present on a crystalline aluminosilicate zeolite, the catalyst having been pretreated with hydrogen to promote selectivity.

U.S. Patent No. 3,647,916 describes an isoparaffin-olefin alkylation process featuring the use of an ion-exchanged crystalline aluminosilicate, isoparaffin/olefin molar ratios below 3:1 and regeneration of catalyst.

U.S. Patent No. 3,655,813 discloses a process for alkylating C₄-C₅ isoparaffins with C₃-C₉ olefins using a crystalline aluminosilicate zeolite catalyst wherein a halide adjuvant is employed in the alkylation reactor. The isoparaffin and olefin are introduced into the alkylation reactor at specified concentrations and catalyst is continuously regenerated outside the alkylation reactor.

U.S. Patent No. 3,236,671 discloses the use, in alkylation, of crystalline aluminosilicate zeolite having silica to alumina mole ratios above 3 and also discloses the use of various metals exchanged and/or impregnated on such zeolites.

U.S. Patent No. 3,706,814 discloses another zeolite-catalyzed isoparaffin-olefin alkylation process and further provides for the addition of C₅+ paraffins such as Udex raffinate or C₅+ olefins to the alkylation reactor feed.

U.S. Patent No. 3,624,173 discloses the use, in isoparaffin-olefin alkylation, of crystalline aluminosilicate zeolites containing gadolinium.

U.S. Patent No. 3,738,977 discloses alkylation of paraffins with ethylene employing a zeolite catalyst which possesses a Group VII metal component, the catalyst having been pretreated with hydrogen.

U.S. Patent No. 3,917,738 describes a process for alkylating an isoparaffin with an olefin using a solid, particulate catalyst capable of absorbing the olefin. The isoparaffin and the olefin are admixed to form a reactant stream in contact with catalyst particles at the upstream end of an adsorption zone after which the reactants are passed concurrently with the catalyst so that a controlled amount of olefin is adsorbed onto the catalyst before the combination of reactants and catalyst is introduced into an alkylation zone. This controlled olefin adsorption is said to prevent polymerization of the olefin during alkylation.

U.S. Patent No. 4,384,161 describes a process of alkylating isoparaffins with olefins to provide alkylate employing as catalyst a large pore zeolite capable of absorbing 2,2,4-trimethylpentane, e.g. ZSM-4, ZSM-20, ZSM-3, ZSM-18, zeolite Beta, faujasite, mordenite, zeolite Y and the rare earth metal-containing forms thereof, and a Lewis acid such as boron trifluoride, antimony pentafluoride or aluminum trichloride. The use of a large pore zeolite in combination with a Lewis acid in accordance with this patent is reported to greatly increase the activity and selectivity of the zeolite thereby effecting alkylation with high olefin space velocity and low isoparaffin-olefin ratio.

US Patent No. 4,774,364 also discloses a process for alkylating isoparaffins with olefins over a Lewis acid-promoted, large pore zeolite catalyst. However, in this case the deactivated zeolite catalyst is reactivated in an alkyltertiary-alkylether synthesis reactor. In this reactor, a mixture of iso-olefin and methanol is reacted in the presence of the deactivated catalyst to produce the required ether and reactivate the catalyst. The product stream from the ether synthesis reactor is distilled to produce an olefin-rich overhead stream and a bottom stream consisting of methanol and ether, from which the ether can be separated. The overhead stream is fed to the alkylation reactor but, in view of the boiling point difference between the olefin and the methanol, will be substantially free of methanol impurities.

It has now been discovered that in an alkylation process in which an isoparaffin is reacted with an olefin in the presence of a large pore zeolite and a Lewis acid to provide an alkylate product, an improvement in the quality of the alkylate is obtained by carrying out the alkylation in the presence of added water and/or water-producing material.

It is known from British Patent No. 546406 that a water/BF₃ mixture can be used to alkylate isoparaffins with ethylene. However, in this early process the water is added in order to form a liquid BF₃-containing catalyst and no advantage as to alkylate product is either disclosed or envisaged.

Accordingly, the invention resides in a process for alkylating an isoparaffin containing up to 20 carbon atoms with an olefin containing from 2 to 12 carbon atoms comprising contacting the isoparaffin and olefin with a catalyst comprising a large pore zeolite capable of absorbing 2,2,4-trimethylpentane and a Lewis acid at a temperature of -40°C to 500°C and a pressure up to 5000 psig (35000kPa), wherein the molar ratio of the isoparaffin to the olefin is from 1:1 to 50:1 and the contacting takes place in the presence of water.

Zeolites used in the present invention are those which have pores sufficiently large to physically absorb 2,2,4-trimethylpentane, such as for example, ZSM-3, ZSM-4, ZSM-12, ZSM-18, ZSM-20, zeolite Beta, zeolite L, mordenite, faujasite, zeolite Y and the rare earth metal-containing forms of the above. A wide range of silica-to-alumina ratios, e.g., from at least 10:1 to 200:1 and even higher, e.g., approaching infinity, can be used. For purposes of this invention, as well as its variant forms including framework dealuminated zeolite Y, e.g., ultrastable Y (USY) described in US Patent No. 3,393,192 and LZ-210 described in US Patent No. 4,503,023.

The large pore zeolite selected for use in the improved alkylation process of this invention generally can possess an alpha value over a wide range of from less than 1 to over 1000. "Alpha value" or "alpha number" is a measure of zeolite acidic functionality and is more fully described together with details of its measurement in US Patent No. 4,016,218, J. Catalysis, 6, pp. 278-287 (1966) and J. Catalysis, 61, pp. 390-396 (1980). Zeolites of low acidity (alpha values of less than 200) can be achieved by a variety of techniques including (a) synthesizing a zeolite with a high silica/alumina ratio, (b) steaming, (c) steaming followed by dealuminization and (d) substituting framework aluminum with other species. For example, in the case of steaming, the zeolite can be exposed to steam at elevated temperatures ranging from 260° to 650°C (500° to 1200°F) and preferably from 400° to 540°C (750° to 1000°F). This treatment can be accomplished in an atmosphere of 100% steam or an atmosphere consisting of steam and a gas which is substantially inert to the zeolite. A similar treatment can be accomplished at lower temperatures employing elevated pressure, e.g., at a temperature of 180° to 370°C (350° to 700°F) and a pressure 1000 to 20000 kPa (10 to 200 atmospheres). Specific details of several steaming procedures may be gained from the disclosures of U.S. Patent No. 4,325,994, 4,374,296 and 4,418,235. Aside from, or in addition to any of the foregoing procedures, the surface acidity of the zeolite can be eliminated or reduced by treatment with bulky reagents as described in U.S. Patent No. 4,520,221.

In practicing the alkylation process of the present invention, it may be advantageous to incorporate the above-described large pore zeolite into some other material, i.e., a matrix or binder, which is stable under the conditions employed in the process. Useful matrix materials include both synthetic and naturally-occurring substances, e.g., inorganic materials such as clay, silica and/or metal oxides. Such materials can be either naturally-occurring or can be obtained as gelatinous precipitates or gels including mixtures of silica and metal oxides. Naturally-occurring clays which can be composited with the zeolite include those of the montmorillonite and kaolin families, which families includes the sub-bentonites and the kaolins commonly known as Dixie, McNamee-Georgia and Florida clays or others in which the main mineral constituent is haloysite, kaolinite, dickite, nacrite or anauxite. Such clays can be used in the raw state as originally mined or initially subjected to calcination, acid treatment or chemical modification.

In addition to the foregoing materials, the zeolites employed herein can be composited with a porous matrix materials such as carbon, alumina, titania, zirconia, silica, silica-alumina, silica-magnesia, silica-zirconia, silica-thoria, silica-beryllia, silica-titania, etc., as well as ternary oxide compositions, such as silica-alumina-thoria, silica-alumina-zirconia, silica-alumina-magnesia, silica-magnesia-zirconia, etc. The matrix can be in the form of a cogel. The relative proportions of zeolite component and natrix material, on an anhydrous basis, can vary widely with the zeolite content ranging from between 1 to 99 wt%, and more usually in the range of 5 to 90 wt% of the dry composite.

In some cases, it may be advantageous to provide the zeolite component of the alkylation catalyst herein as an extrudate bound with a low acidity refractory oxide binder employing the method described in an International Publication No. WO88/08329. In accordance with said method, a homogeneous mixture of a large pore zeolite such as zeolite Beta, water and a low acidity refractory oxide binder, e.g., silica, which contains at least an extrusion-facilitating amount of the binder provided in a colloidal state and which is substantially free of added alkali metal base and/or basic salt, is formed into an extrudable mass, the mass is extruded and the resulting extrudate is dried and calcined.

The original cations associates with the zeolite utilized herein can be replaced by a wide variety of other cations according to techniques well known in the art, e.g., by ion-exchange. Typical replacing cations include hydrogen, ammonium, alkyl ammonium and metal cations, and their mixtures. In the case of metal cations, particular preference is given to such metals as magnesium, zinc, calcium, zinc, and mixtures thereof. A typical ion-exchange technique involves contacting the particular zeolite with a salt of the desired replacing cation. Although a wide variety of salts can be employed, particular preference is given to chlorides, nitrates and sulfates. Representative ion-exchange techniques are disclosed in a number of patents including U.S. Patent Nos. 3,140,249; 3,140,251; 3,140,253 and 3,702,886.

Following contact with a solution of the desired replacing cation, the zeolite is then preferably washed with water and dried at a temperature ranging from 65° to 315° (150° to 600°F) and thereafter calcined in air of inert gas at a temperature ranging from 260° to 815° (500° to 1500°F) for periods of time ranging from 1 to 48 hours or more.

As previously stated, the alkylation catalyst employed herein comprises a large pore zeolite in combination with a Lewis acid. A Lewis acid is generally considered to be a molecule which is capable of combining with another molecule or ion by forming a covalent chemical bond with two electrons from the second molecule or ion, that is to say, the Lewis acid is an electron acceptor. Examples of Lewis acids include boron trifluoride (BF₃) antimony pentafluoride (SbF₅) and aluminum chloride (AlCl₃). The present invention contemplates the use of all Lewis Acids such as those set forth in "Friedel-Crafts and Related Reactions", Interscience Publishers, Chapters III and IV (1963), although boron trifluoride is preferred. In general, the Lewis acid will be present in the alkylation zone in an amount which exceeds that required to saturate said zeolite catalyst composition considered not only as the zeolite per se but as any other material, e.g., binder or matrix materials, which might be associated with the zeolite. Thus, the reaction environment will contain BF₃ over and above the amount sorbed or taken up by the zeolite and any binder with which the zeolite might be composited.

The operating temperature of the alkylation process herein can extend over a fairly broad range, i.e., from -40° to 500°C, preferably -40° to 250°C, and more preferably -40° to 40°C. The practical upper operating temperature will often be dictated by the need to avoid an undue occurrence of undesirable side reactions.

The pressures employed in the present process can also extend over a wide range, e.g., from subatmospheric to 35000 kPa (5000 psig), preferably to 3600 kPa (500 psig).

Similarly the amount of catalyst used in the present process can vary over relatively wide limits. In general, the amount of catalyst as measured by the weight hourly space velocity of the olefin can range from 0.01 to 100. It will, of course, be realized by those skilled in the art that the amount of catalyst selected for a particular reaction will be determined by several variables including the reactants involved as well as the nature of the catalyst and the operating conditions employed.

The isoparaffin reactant used in the present alkylation process possesses up to 20 carbon atoms and preferably from 4 to 8 carbon atoms such as, for example, isobutane, 3-methylhexane, 2-methylbutane, 2,3-dimethylbutane and 2,4-dimethylhexane.

The olefin reactant employed herein generally contains from 2 to about 12 carbon atoms. Representative examples are ethylene, propylene, butene-1, butene-2, isobutylene, pentenes, hexenes, heptenes and octenes. Particularly preferred are C₃ and C₄ olefins and mixtures thereof. Conveniently the olefinic feed used in the present process is first contacted with an isomerization catalyst to reduce its alpha-olefin content. Isomerization catalysts which can be used in the isomerization operation include catalysts which produce a shift of the double bond in 1-butene to a more central position in the hydrocarbon molecule to form 2-butene. Various conventional catalysts are suitable, including, for example, alumina, silica, zirconia, chromium oxide, boron oxide, thoria, magnesia, aluminum sulfate, and combinations thereof. Also suitable is a boron halide-modified metal oxide such as boron halide-modified substantially anhydrous or hydrous alumina. Thermal isomerization may be utilized, but suffers from the defect of producing excessive amounts of side products. The isomerization step may also include a separation section to remove unconverted 1-butene, which can then be recycled to the isomerization reactor. Alkylation follows isomerization.

In general, the relative molar ratio between the isoparaffin reactant and the olefin alkylating agent in the combined hydrocarbon feed is from 1:1 to 50:1, preferably from 5:1 to 25:1.

A critical requirement of the present alkylation process is that water is introduced into the alkylation reactor, e.g., at a rate of from 0.1 ppmw to 1 wt%, based upon total hydrocarbon feed rate, preferably at a rate of from 0.1 ppmw to 500 ppmw. The water can be supplied as such or as a feed material which provides water under the alkylation conditions selected. Suitable water-forming materials which can be introduced into the reactor without interfering with the desired alkylation include monohydric and dehydric alcohols which yield water upon undergoing dehydration. Of this group, particular preference is accorded the aliphatic alcohols, especially those containing 1 to 6 carbon atoms such as, for example, methanol, ethanol, isopropanol, t-butyl alcohol and isopentyl alcohol. The water and/or water-producing material can be added directly to the reactor, e.g., as part of the feed and/or can be incorporated in the zeolite, either by direct contact therewith or by exposing the zeolite to an atmosphere of water and/or water-forming material. For example, when the added water and/or water-forming material is preintroduced into the zeolite catalyst, the amount of water and/or water-forming materials taken up by the catalyst can vary from 0.5 to 25, and preferably from 1 to 10, weight percent of the catalyst.

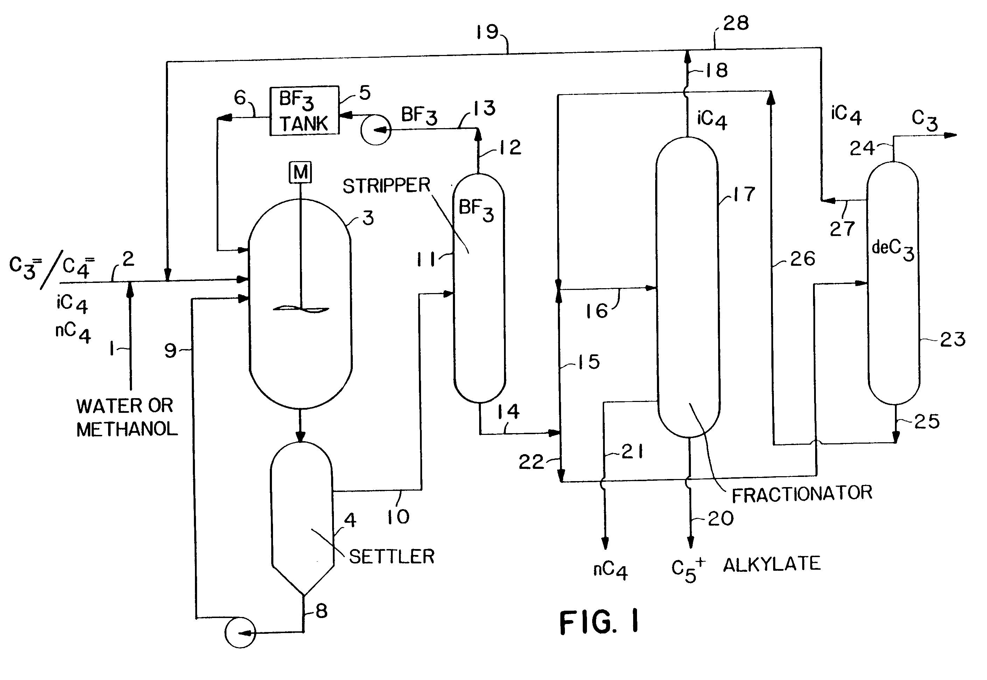

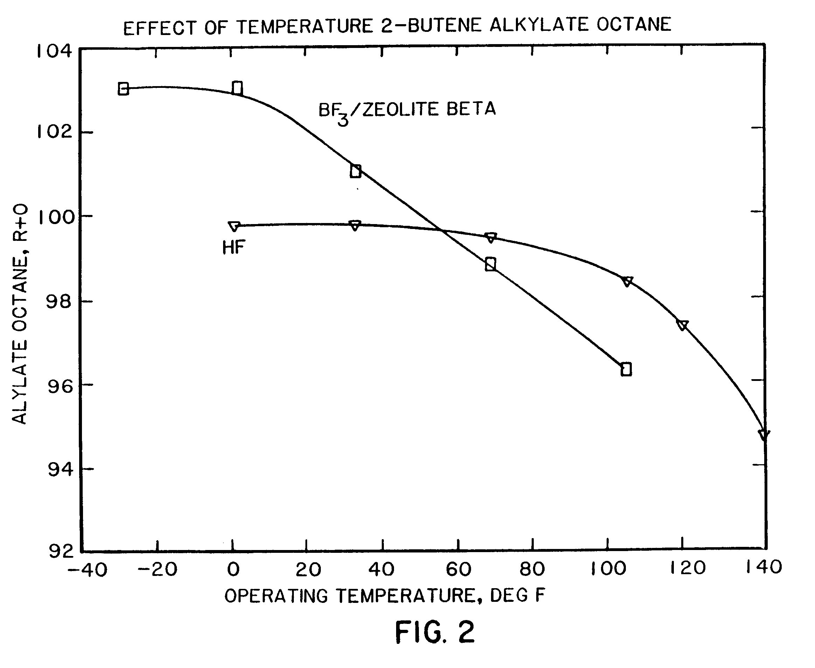

The invention will now be more particularly described with reference to the accompanying drawings, in which Figure 1 a schematic representation of a system for effecting an alkylation process according to one example of the invention, and Figure 2 illustrates graphically the effect of temperature on octane for alkylation of isobutane with 2-butene for BF₃/zeolite beta catalyst of the invention and for a conventional HF catalyst.

Referring to the drawing, in the system shown water and/or a water-producing material such as methanol is introduced through line 1 to a stream containing isobutane and propylene and/or butenes along with some normal butane introduced through line 2 to stirred reactor 3 containing a zeolite catalyst. BF₃ is introduced as needed from a holding tank 5 through line 6 into the reactor 3. The amount of BF₃ introduced is such as to exceed the amount required to saturate the zeolite catalyst (as well as any binder or matrix material with which the zeolite might be composited). Catalyst slurry is removed from the reactor and is introduced to settling vessel 4, the recovered zeolite catalyst thereafter being recycled to the alkylation reactor via line 9. The hydrocarbon product mixture is removed from the settling vessel 4 through line 10 and introduced into BF₃ stripper 11 from which BF₃ is removed as overhead through line 12 and recycled through line 13 to BF₃ holding tank 5. The remaining hydrocarbon product mixture is withdrawn from the BF₃ stripper through line 14. A portion of such hydrocarbon product mixture is introduced via lines 15 and 16 to fractionator 17. Unreacted isobutane is removed as overhead through line 18 and recycled through line 19 to the reactant feed stream line 2. Desired C₅+ alkylate product is withdrawn from the bottom of fractionator 17 through line 20. Any normal butane may be withdrawn from the fractionator through line 21. The remaining portion of the hydrocarbon product mixture passing through line 14 from BF₃ stripper 11 is conducted through line 22 to depropanizer 23 from which propane and lighter products are removed as overhead through line 24. Heavier components are removed as bottoms through line 25 and recycled via lines 26 and 16 to fractionator 17. Isobutane is removed from depropanizer 23 through line 27 and recycled through lines 28 and 19 to the initial reactant feed line 2.

The following Examples serve to illustrate the process of the invention.

### EXAMPLE 1

Two unbound zeolite Beta catalysts containing 6 and 16 weight percent water, respectively, were employed in the alkylation of isobutane with butene-1. The varying water content was obtained by exposing separate portions of the zeolite catalyst to low and high moisture content atmospheres.

In each case, 10 grams of zeolite Beta (100% solids basis) was placed in a 300 ml autoclave reactor, and about 300 ml of isobutane was charged to fill the reactor. The resulting mixture was cooled to 0°C with constant stirring at 1900 RPM and BF₃ gas was introduced into the reactor. After BF₃ breakthrough was observed, the BF₃ flow rate was reduced to a level equivalent to 3 wt% of total hydrocarbon feed rate. At this point, a 10/1 isobutane/butene-1 mixture was continuously fed into the reactor to initiate the catalytic alkylation. The operating conditions were 1135 kPa (150 psig), 0°C, 1900 RPM, 1.2 WHSV based on olefin and 3.0 wt% BF₃ based on total hydrocarbon feed. The product was continuously withdrawn from the reactor and was adjusted to atmospheric pressure via a back pressure regulator and then sent to a receiver which was kept at 0°C. Periodically, the product was drained from the receiver and allowed to reach room temperature prior to analysis.

An on-line gas chromatograph coupled with an automatic sampling device was used to monitor the course of the alkylation reaction. All reported clear octane numbers were measured.

Tables 1 and 2 show the yield/octane results for the low and high water-containing zeolite Beta catalysts, respectively. In both cases the C₅+ yield data indicate that alkylation was essentially complete. As the data show, a significant improvement in the alkylate quality was observed for the 16 weight percent water-content zeolite Beta catalyst. Specifically, the octane numbers from the high water-content catalyst were substantially higher over the course of the experiment. In addition, the C₉+ content of the alkylate product was reduced with the higher moisture content catalyst.

**TABLE 1**

| Performance of "Low Water Content" Zeolite-Beta (6 wt.% H₂0) For Isobutane/Butene-1 Alkylation | | | |
|---|---|---|---|
| Time on Stream, hr. | 4 | 10 | 20 |
| Yield, g C₅+/g Olefin Converted | 2.1 | 2.3 | 2.2 |

| Yields in C₅+, wt% | | | |
|---|---|---|---|
| C₅-C₇ | 0.6 | 1.0 | 1.7 |
| C₈ | 88.2 | 89.9 | 86.4 |
| C₉+ | 11.2 | 9.1 | 11.9 |

| Octane | | | |
|---|---|---|---|
| RON+O | 71.0 | 71.8 | 67.3 |
| MON+O | 76.1 | 77.6 | 70.3 |

**TABLE 2**

| Performance of "Higher Water Content" Zeolite-Beta (16 wt.% H₂0) For Isobutane/Butene-1 Alkylation | | | |
|---|---|---|---|
| Time on Stream, hr. | 4 | 8 | 18 |
| Yield, g C₅+/g Olefin Converted | 2.2 | 2.3 | 1.9 |

| Yields in C₅+, wt% | | | |
|---|---|---|---|
| C₅-C₇ | 1.8 | 0.7 | 1.5 |
| C₈ | 90.3 | 95.9 | 94.4 |
| C₉+ | 7.9 | 3.4 | 4.1 |

| Octane | | | |
|---|---|---|---|
| RON+O | 77.1 | 79.3 | 86.0 |
| MON+O | 80.1 | 80.9 | 81.4 |

## Claims

1. A process for alkylating an isoparaffin containing up to 20 carbon atoms with an olefin containing from 2 to 12 carbon atoms comprising the step of contacting the isoparaffin and olefin in a reaction zone with a catalyst comprising a large pore zeolite capable of absorbing 2,2,4-trimethylpentane and a Lewis acid at a temperature of -40°C to 500°C and a pressure up to 500 psig (3500 kPa), wherein the molar ratio of the isoparaffin to the olefin is from 1:1 to 50:1, characterized by comprising the additional step of adding water or a water producing compound to the reaction zone during said contacting step.

2. The process of Claim 1, wherein the weight hourly space velocity of the olefin is from 0.01 to 100.

3. The process of Claim 1, wherein the isoparaffin contains from 4 to 6 carbon atoms and the olefin contains from 2 to 6 carbon atoms.

4. The process of Claim 1, wherein the Lewis acid is BF₃, BCl₃, SbF₅ and/or AlCl₃.

5. The process of Claim 1, wherein the Lewis acid is BF₃.

6. The process of Claim 1, wherein the zeolite is selected from ZSM-3, ZSM-4, ZSM-12, ZSM-18, ZSM-20, zeolite Beta, zeolite L, mordenite, faujasite and zeolite Y.

7. The process of Claim 1, wherein the catalyst is BF₃/zeolite Beta.

8. The process of Claim 1, wherein the amount of water ranges from 0.1 ppmw to 1 weight percent based upon the total hydrocarbon feed rate.

9. The process of Claim 1, wherein reaction temperature is from -40°C to 15°C.

10. The process of Claim 1, and including the step of subjecting the olefin feed to an initial isomerization treatment to reduce the alpha olefin content thereof.

## Patentansprüche

1. Verfahren zur Alkylierung eines Isoparaffins, das bis zu 20 Kohlenstoffatome enthält, mit einem Olefin, das 2 bis 12 Kohlenstoffatome enthält, welches den Schritt umfaßt: Kontakt des Isoparaffins und des Olefins in einer Reaktionszone mit einem Katalysator, der einen großporigen Zeolith, der 2,2,4-Trimethylpentan absorbieren kann, und eine Lewis-Säure umfaßt, bei einer Temperatur von -40 bis 500°C und einem Druck bis zu 500 psig (3.500 kPa) wobei das Molverhältnis von Isoparaffin zum Olefin 1:1 bis 50:1 beträgt, dadurch **gekennzeichnet,** daß es den zusätzlichen Schritt der Zugabe von Wasser oder einer wassererzeugenden Verbindung zur Reaktionszone während des Kontaktschritts umfaßt.

2. Verfahren nach Anspruch 1, wobei die stündliche Gewichts-Raum-Geschwindigkeit des Olefins 0,01 bis 100 beträgt.

3. Verfahren nach Anspruch 1, wobei das Isoparaffin 4 bis 6 Kohlenstoffatome und das Olefin 2 bis 6 Kohlenstoffatome enthält.

4. Verfahren nach Anspruch 1, wobei die Lewis-Säure BF₃, BCl₃, SbF₅ und/oder AlCl₃ ist.

5. Verfahren nach Anspruch 1, wobei die Lewis-Säure BF₃ ist.

6. Verfahren nach Anspruch 1, wobei der Zeolith aus ZSM-3, ZSM-4, ZSM-12, ZSM-18, ZSM-20, Zeolith Beta, Zeolith L, Mordenit, Faujasit und Zeolith Y ausgewählt ist.

7. Verfahren nach Anspruch 1, wobei der Katalysator BF₃/Zeolith Beta ist.

8. Verfahren nach Anspruch 1, wobei die Wassermenge im Bereich von 0,1 Gewichtsteil pro Million bis 1 Gew.-% auf der Basis der Gesamtmenge der Kohlenwasserstoffbeschickung liegt.

9. Verfahren nach Anspruch 1, wobei die Reaktionstemperatur -40 bis 15°C beträgt.

10. Verfahren nach Anspruch 1, das den Schritt der anfänglichen Isomerisierungsbehandlung der Olefinbeschickung umfaßt, damit deren Gehalt an α-Olefinen verringert wird.

## Revendications

1. Procédé d'alkylation d'une isoparaffine contenant jusqu'à 20 atomes de carbone par une oléfine comportant de 2 à 12 atomes de carbone, comprenant une étape de mise en contact de l'isoparaffine et de l'oléfine, dans une zone de réaction, avec un catalyseur comprenant une zéolite à larges pores capable d'absorber le 2,2,4-triméthylpentène et un acide de Lewis, à une température comprise entre -40°C et 500°C et sous une pression pouvant atteindre 3500 kPa (500 psig), dans lequel le rapport molaire de l'isoparaffine à l'oléfine est compris entre 1:1 et 50:1, caractérisé en ce qu'il comprend l'étape supplémentaire d'addition d'eau ou d'un composé libérant de l'eau dans la zone de réaction pendant cette étape de mise en contact.

2. Procédé selon la revendication 1, dans lequel la vitesse horaire spatiale pondérale de l'oléfine est comprise entre 0,01 et 100.

3. Procédé selon la revendication 1, dans lequel l'isoparaffine comporte de 4 à 6 atomes de carbone et l'oléfine comporte de 2 à 6 atomes de carbone.

4. Procédé selon la revendication 1, dans lequel l'acide de Lewis est BF₃, BCl₃, SbF₅ et/ou AlCl₃.

5. Procédé selon la revendication 1, dans lequel l'acide de Lewis est BF₃.

6. Procédé selon la revendication 1, dans lequel la zéolite est choisie dans le groupe consistant en ZSM-3, ZSM-4, ZSM-12, ZSM-18, ZSM-20, zéolite Bêta, zéolite L, mordénite, faujasite et zéolite Y.

7. Procédé selon la revendication 1, dans lequel le catalyseur est constitué de BF₃/zéolite Bêta.

8. Procédé selon la revendication 1, dans lequel la quantité d'eau est comprise entre 0,1 ppm en poids et 1% en poids par rapport au débit d'alimentation de la charge hydrocarbonée totale.

9. Procédé selon la revendication 1, dans lequel la température de réaction est comprise entre -40°C et 15°C.

10. Procédé selon la revendication 1, et comprenant l'étape de soumission de la charge d'oléfines à un traitement préalable d'isomérisation pour abaisser la teneur en alpha-oléfines de la charge.
